Europäisches Patentamt

European Patent Office

Office européen des brevets

(11) Veröffentlichungsnummer: **0 320 625 B1**

(12) **EUROPÄISCHE PATENTSCHRIFT**

(45) Veröffentlichungstag der Patentschrift: **14.09.94**

(21) Anmeldenummer: **88118808.0**

(22) Anmeldetag: **11.11.88**

Die Akte enthält technische Angaben, die nach dem Eingang der Anmeldung eingereicht wurden und die nicht in dieser Patentschrift enthalten sind.

(51) Int. Cl.5: **A61L 9/00**

(54) **Riechstoffverwendung, insbesondere beim Staubsaugen.**

(30) Priorität: **15.12.87 DE 3742477**
**14.07.88 DE 3823807**

(43) Veröffentlichungstag der Anmeldung:
**21.06.89 Patentblatt 89/25**

(45) Bekanntmachung des Hinweises auf die Patenterteilung:
**14.09.94 Patentblatt 94/37**

(84) Benannte Vertragsstaaten:
**AT BE CH DE ES FR GB GR IT LI LU NL SE**

(56) Entgegenhaltungen:
**EP-A- 0 031 455**
**WO-A-81/00051**
**DE-A- 3 404 395**
**FR-A- 2 520 603**

(73) Patentinhaber: **Vorwerk & Co. Interholding GmbH**
**Mühlenweg 17-35**
**D-42275 Wuppertal (DE)**

(72) Erfinder: **Polligkeit, Wolfgang, Dr.**
**Wuppermannstrasse 99**
**D-5828 Ennepetal (DE)**
Erfinder: **Neugart, Horst**
**Schmitteborn 147**
**D-5600 Wuppertal 22 (DE)**

(74) Vertreter: **Müller, Enno et al**
**Postfach 11 04 51**
**D-42304 Wuppertal (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 320 625 B1

**Beschreibung**

Die Erfindung betrifft die Verwendung von Riechstoffgemischen, die einem Luftstrom beizugeben sind, der elektrostatisch aufgeladene Filter durchströmt. Insbesondere betrifft die Erfindung die Beigabe von Riechstoffgemischen zu einem Luftstrom, welcher derartige Filter in einem Staubsauger durchströmt.

Bei bekannten Staubsaugern wird eine Filtration der staubbeladenen angesaugten Luft zunächst im Staubsaugerbeutel durchgeführt. Nachgeschaltet zu dem Staubsaugerbeutel ist vielfach ein elektrostatisch aufgeladenes Filter, welches verbleibende Feinstäube ausfiltern soll. Gleichwohl ist festzustellen, daß die aus dem Staubsauger austretende Luft einen gewissen Staubgeruch verbreitet, obwohl praktisch alle Staubpartikel abgeschieden sind. Um dem zu begegnen, ist man bereits dazu übergegangen, Riechstoffe in Form von Stäbchen oder dergleichen z. B. in den Staubsaugerbeutel einzulegen. Die durchströmende Luft reißt Riechstoffpartikel mit, die helfen, in der aus dem Staubsauger austretenden Luft einen Staubgeruch zu überdecken. Solche mit Riechstoffen betriebenen Staubsauger mit nachgeschaltetem elektrostatisch aufgeladenem Filter werden in DE-A 3 404 495 beschrieben. Es wurde gefunden, daß bei derartig betriebenen Staubsaugern die Filtriereigenschaften des elektrostatischen Filters relativ rasch nachlassen. Der Dufteindruck bleibt im wesentlichen unverändert, aber der Feinstaubanteil steigt.

Aus der FR-A 2 520 603 ist es für sich bekannt, in einem Staubsauger eine Desodorierungsvorrichtung vorzusehen. Aus der EP-A 0 031 455 schließlich ist eine pulvrige Riechstoff-Zusammensetzung zu Luftverbesserung beim Reinigen von Teppich bekannt, die zusammengesetzt ist aus anorganischen Salzen, Riechstoffen und Zeolithen, wobei die Riechstoff-Zusammensetzungen nur allgemein angegeben sind, nicht aber etwa hinsichtlich bestimmter molekularer Eigenschaften ausgewählt.

Die elektrosatisch aufgeladenen Filter sind bevorzugt sogenannte Electretfilter. Bei einem Electret handelt es sich beispielsweise um Vliese, deren Fasern durch geeignete Vorbehandlung permanente positive und/oder negative Ladungen enthalten. Diese Ladungen ziehen die ebenfalls aufgeladenen oder induzierten Feinstaubpartikel an und halten sie elektrostatisch fest. Als geeignete Fasermaterialien sind solche auf Basis von Polypropylen (PP) und Polycarbonat (PC) bekannt. Je nach Herstellverfahren und Behandlungsverfahren der Fasern ergeben sich unterschiedliche Eigenschaften hinsichtlich Staubkapazität, Abscheidegrad und Filterwiderstand der Electrete.

Im Hinblick auf die beschriebenen Unzulängigkeiten stellt sich der Erfindung die Aufgabe, bei möglichst unverändert guter Überdeckung des Staubgeruches der aus dem Staubsauger austretenden Luft die Filtriereigenschaften langfristig auf gleichem Niveau zu halten.

Diese Aufgabe ist nach der Lehre der Erfindung gelöst durch die Verwendung von polaren, aprotischen Riechstoffen mit einem Molekulargewicht zwischen 100 und 300 zur Anreicherung von durch elektrostatisch aufgeladene Filter auf Basis von Polypropylen strömende Luft. Die Aufgabe ist desgleichen gelöst durch die Verwendung apolarer, aprotischer (unpolar) Riechstoffe mit einem Molekulargewicht zwischen 100 und 300, zur Anreicherung von durch elektrostatisch aufgeladene Filter aus der Basis von Polycarbonat strömende Luft. Überraschenderweise hat sich gezeigt, daß praktisch über die gesamte Betriebsdauer des Filters unverändert gute Dufteigenschaften erhalten werden, bei gleichzeitiger Aufrechterhaltung der optimalen Feinstaub-Filtriereigenschaften.

Derartige Riechstoffe, wie sie vorstehend angesprochen sind, können dem Staubsauger durch Einlegung in den Staubsaugerbeutel zunächst in an sich bekannter Form, in Form von Stäbchen, beigegeben werden. Eine besondere Lehre der Erfindung geht jedoch dahin, die Riechstoffe dem Flächenpflegemittel (für Böden, Polster etc.), mit welchem eine Fläche zur Aktivierung vor dem Staubsaugen behandelt wird, beizugeben. Hiermit ist es nicht mehr erforderlich, gesondert derartige Stäbchen od. dgl. in den Staubsaugerbeutel einzulegen. Gleichsam automatisch werden beim Staubsaugen die Partikel mit dem Flächenpflegemittel eingesaugt und durchsetzen den Staubsauger, um schließlich in der vom Staubsauger abgegebenen, gefilterten Luft für eine duftmäßige Überdeckung des möglichen Staubgeruches zu sorgen. Die Erfindung lehrt, ein Reinigungsmittel für eine dem Staubsaugen vorausgehende Flächenbehandlung zu verwenden, welches Riechstoffe enthält mit einem Molekulargewicht zwischen 100 und 300, die entweder vorwiegend polar und aprotisch oder vorwiegend apolar und aprotisch sind. Die Reinigungsmittel mit vorwiegend polar und aprotischen Riechstoffen sind zu verwenden, wenn beim nachfolgenden Staubsaugen Filter auf der Basis von Polypropylen eingebaut sind. Entsprechend ist ein Reinigungsmittel mit Riechstoffen, die vorwiegend apolar und aprotisch sind zu verwenden, wenn beim nachfolgenden Staubsaugen Filter auf der Basis von Polycarbonat zur Anwendung kommen.

Im Einzelnen ergibt sich hinsichtlich der Riechstoffe eines Riechstoffgemisches, das bei einem PP-Electret vor allem azyklische, monozyklische und bizyklische Terpinkohlenwasserstoffe und davon abgeleitete Alkohole, Ester, Aldehyde und Ketone absorbiert werden, je nach Typ unterschiedlich stark. Besonders stark werden hierbei als einzelne Riechstoff Pinene, Myrcen, Phellandren, Ocimen, Limonen und Cineol

2

absorbiert, weniger stark jedoch Linalool und Terpinylacetat, nicht absorbiert wird dagegen Methyljonon.

Kaum oder gar nicht werden dagegen wichtige Riechstoffe absorbiert, die von polaren, aliphatischen Aldehyden abgeleitet sind. Desgleichen werden wenig oder kaum absorbiert aliphatische Carbonsäureestern, aliphatisch-aromatische Estern oder aromatische Aldehyde. Als Beispiele für die zuvorgenannten Gruppen sei genannt Amylzimtaldehyd, Alkylacetat, Salicylsäureamylester, Propionsäurebenzylester und Heliatropin. Stark polare, wasserlösliche organische Hilfsstoffe wie Ethanol und Isopropanol werden überhaupt nicht absorbiert.

Ein Electret aus Basis von Polycarbonat ist gut beständig gegenüber Terpentin (beispielsweise Pinene, Phellandren), nicht beständig jedoch gegenüber aliphatischen und aromatischen Estern. Unter letztere fällt z.B. Butylacetat und Salicylsäureethylester.

Beispiel eines polaren, aprotischen Duftstoffes zur Verwendung bei Staubsaugern

| Parfümöl F 45 | | |
|---|---|---|
| Methylnaphthylketon | 10 % DPG | 20 |
| Ambroxane | 1 % IPM | 10 |
| Calone | 1 % DPG | 10 |
| Isobutylchinaline | 1 % DPG | 10 |
| Dihydrojasmin | 1 % DPG | 10 |
| Galbex | 1 % DPG | 20 |
| Mandarinal | 10 % DPG | 3 |
| Methylantranilsäure-Methylester | 10 % DPG | 3 |
| Indolal | 10 % DPG | 7 |
| Nercelin Bramilia | 10 % DPG | 5 |
| Celestolide | 10 % DPG | 5 |
| Isoeugenol | 10 % DPG | 10 |
| Farenal | 10 % DPG | 10 |
| Cuminaldehyd | 10 % DPG | 3 |
| Benzoesäure-Methylester | 10 % DPG | 7 |
| Elintaal | 10 % DPG | 3 |
| Canangaöl Java | | 3 |
| Base Flieder Spezial 0/222257 | | 100 |
| Orangenöl guinea süss | | 10 |
| Base Mandarine 0/230380 | | 15 |
| Geronial/Nerol-Gemisch | | 10 |
| Grenylisobutyrol | | 5 |
| Benzylsalizilat | | 70 |
| Hunylsalizilat | | 15 |
| Galaxolide 50 | | 45 |
| Tonalide | | 5 |
| Base Lemon Blossom spezial VK 224 | | 300 |
| Base Blumengrün KU 104/A1 | | 170 |
| Chrysanthemengrün AP 2244 forte | | 116 |
| | | 1000 |

Bei den oben erwähnten Abkürzungen "DPG" und "IPM" handelt es sich um an sich bekannte Lösungsmittel. "DPG" bezeichnet etwa Dipropylenglykol.

Mit einem wie vorstehend spezifizierten Parfümöl sind in einem wie weiter unten noch erläuterten Versuch folgende Werte ermittelt worden:

| Parfümöltyp | F 45 |
|---|---|
| Untersuchungsmerkmal | |
| GC-Methode Dani Nr. | 34 |
| GC-Nr. Säulentemp ° C | 852 I |
| Probegastemp. ° C | 20 |
| Peakfläche ohne Electret | 49377 |
| Peakfläche mit Electret | 19881 |
| Flächenverlust % | 60 |
| Einwirkdauer** | |
| Duftstoff auf Vlies [h] | 93 |
| Duftstoffmenge mg auf Filter ⌀ | 28 |
| Partikelabscheidegrad Electret % | 93,8 |
| Partikelabscheidegrad Electret mit Duftstoff % | 96,2 |
| Verschlechterung der Filterwirkung % | + 1,4 |

Bei der vorstehenden Tabelle bedeutet "GC": Gaschromatograph. Bei der Methode 34 werden 1g und bei den Methoden 36 bzw. 38 2g Parfümöl in eine 500ml fassende Schraubglasflasche eingewogen und durch ein alu-kaschiertes Septum und eine durchbohrte Schraubkappe verschlossen. Die Kurve der Gaschromatographen-Nummer 852 I ist in der Anlage beigefügt. Bei der Säule handelt es sich um eine 30m Kapillarsäule DB 1 unpolar (SE 30). Bei der Gasprobenentnahme wird so vorgegangen, daß aus dem Dampfraum der weiter oben erwähnten Flasche bei 40 ° Celsius 0,6 ml mit einer auf 40 ° Celsius temperierten gasdichten Glasspritze SGE/10ml entnommen wird, in den Injektor des Gaschromatographen injiziert wird und sodann die Analyse gestartet wird. Bezüglich der Adsorption wird in dieselbe, erwähnte Spritze einlagig ausgestanztes Electretfilter (⌀ 18 mm, 40 mg) eingesetzt und mit dem Spritzenkolben auf den Spritzboden gedrückt, das ablesbare Filtervolumen beträgt 0,2 ml. Die so vorbereitete Spritze wird im Trockenschrank auf 40° Celsius temperiert.

Gasproben-Entnahme und Adsorption:

Aus dem Dampfraum der Flasche wird wieder 0,6 ml Dampf entnommen, indem der Kolben bis zur Marke 0,8 ml (0,2 ml Filter-Volumen + 0,6 ml Dampf) gezogen wird (1. Filtration). Bei der sofort folgenden Injektion wird der Parfümöldampf nochmals durch das Electretfilter gedrückt (2. Filtration).

Die Aufzeichnung und Auswertung des Gaschromatographen wird mittels des Integrator C-R1B nach Methode 41 durchgeführt:

Normalintegration des einzelnen Peaks sowie der Gesamtfläche ohne jegliche Faktorisierung. Hinsichtlich der Geräte und Hilfsmittel zur gaschromatograpohischen Untersuchung ist weiter unten noch eine Aufstellung aufgeführt.

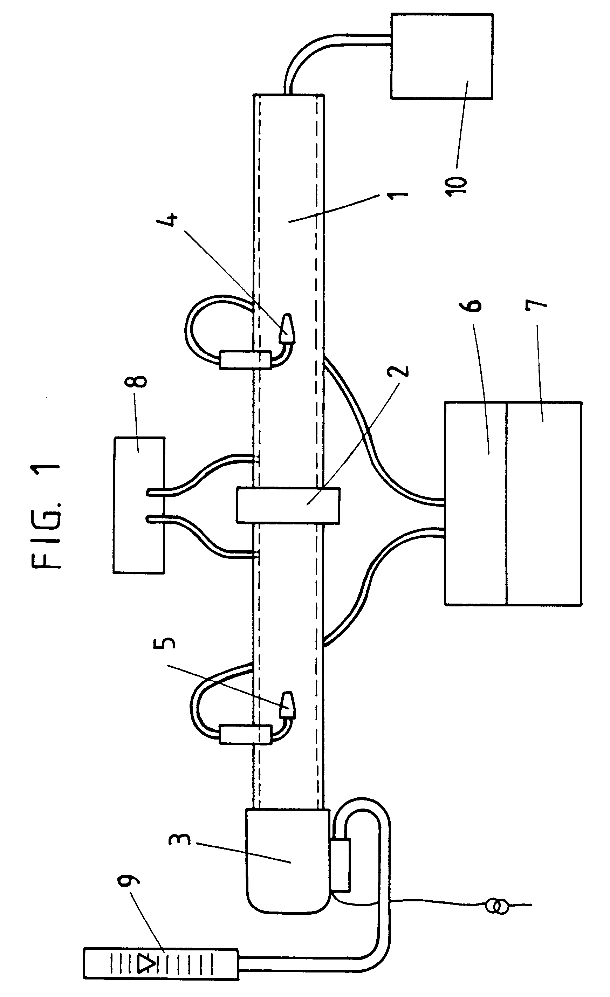

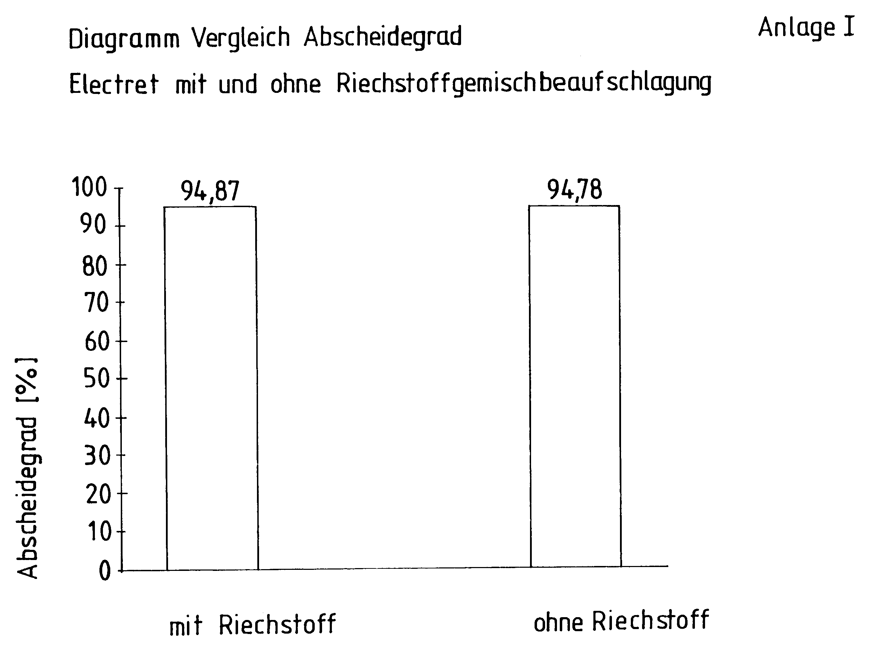

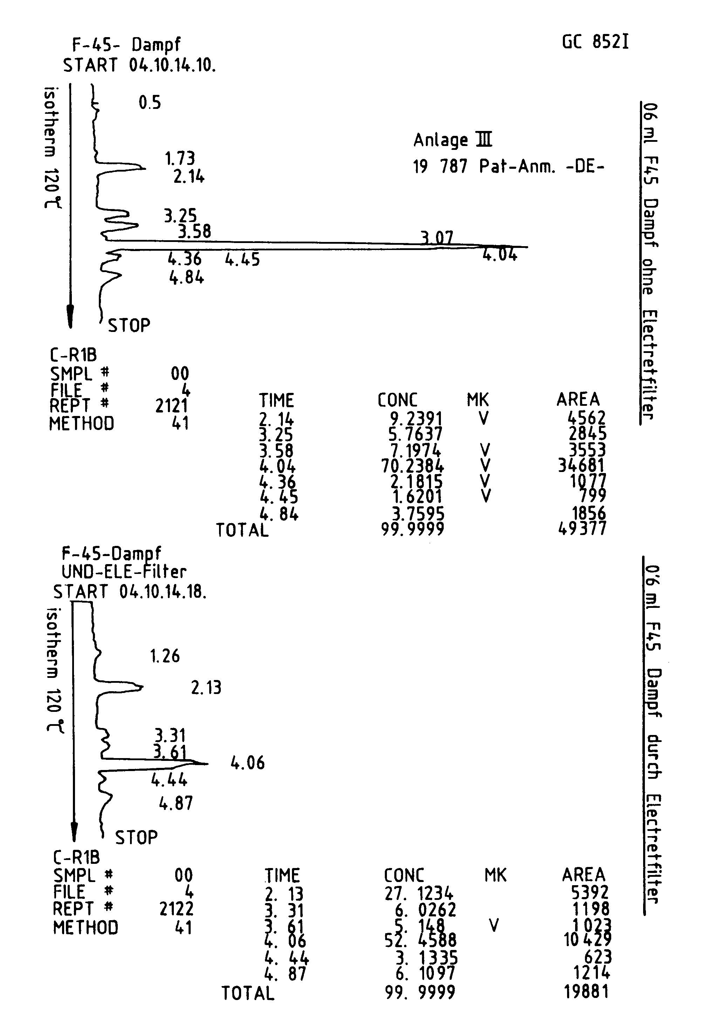

Bezüglich der Gesamtversuchsanordnung wird auf die beigefügte Zeichnung verwiesen, auf welcher folgender Aufbau der Filterprüfmeßstrecke zur Ermittlung des Abscheidegrades zu erkennen ist:

Die komplett geerdete Meßstrecke besteht aus einem Plexiglasrohr 1 von ca. 2m Länge und 0,15m Durchmesser, welches im Abstand von 1,2m vom Rohranfang eine Aufnahme 2 für ein Prüffilter besitzt. Am Ende der Meßstrecke ist ein Motorgebläse 3 mit stufenloser Regelung angeordnet. Vor und nach der Aufnahme 2 für das Prüffilter sind jeweils Meßsonden 4, 5 angebracht, die zur Teilvolumenstromentnahme dienen. Die entnommenen Teilvolumenströme werden zu dem Gassensor 6 und dem Partikelzähler 7 geleitet.

Das Motorgebläse 3 wird mittels eines zwischen 0 und 220 Volt steuerbaren Transformators gesteuert. Mit 8 ist eine Differenzdruckmessung bezeichnet und mit 9 eine Volumenstrommessung. 10 bezeichnet einen Staubgenerator.

Die Filterscheiben mit einem Durchmesser von 162mm werden aus einer Rollenware von Electretvlies (Firma 3M, nach dortigen Angaben gemäß deutschem Patent 25 12 885) mit einem Kreisschneider ausgeschnitten.

Die Behandlung der Proben mit den Riechstoffgemischen erfolgt in einem Exsikkator. Dessen Boden wird mit einer ausreichenden Menge Parfümöl bedeckt. Im Gasraum darüber werden mindestens 3 Scheiben/Parfümöl in einem Gestell aufgehängt. Zur Beschleunigung der Adsorption des Filters mit Parfümöl wird die Atmosphärenluft im Exsikkator kurz abgepumpt (auf etwa $10^2$ Millibar). Der verschlossene

Exsikkator wird mit den Proben bei Raumtemperatur (20° Celsius) bis zur Messung 48 Stunden gelagert.

Bei der Filterabscheidegrad-Messung wird derart vorgegangen, daß vor Meßbeginn die parfümierten Filterscheiben in die Püffilter-Aufnahme eingesetzt werden. Dann wird der Staubgenerator mit voller Leistung und dem 5er Staubkonus bei 121/min und 7mm Hubvorschub betrieben. Der dabei freigesetzte Prüfstaub wird in die Meßstrecke eingeblasen. Nun werden mit dem Partikelzählgerät jeweils 30 Sekunden lang Teilluftvolumenströme vor und nach dem Prüfling entnommen und die darin enthaltenen Staubpartikel zwischen 0,3 bis 20µm Partikeldurchmesser ausgezählt. Dies erfolgt dreimal aus statistischen Gründen, wobei die Meßergebnisse sodann ausgedruckt werden. Die gemessenen Partikelzahlen vor und nach dem Prüfling werden umgekehrt proportional ins Verhältnis gesetzt und so als Abscheidegrad in Prozent ausgedrückt.

Angesichts der Tatsache, daß, gemessen am Staubrückhaltevermögen solcher aus Polypropylen bestehender elektrostatischer Filter, praktisch nur polare und aprotische Substanzen, des Molekulargewichtes zwischen 100 und 300, vorhanden sind - filterblockierende Substanzen unpolaren und protischen Charakters also praktisch fehlen - , ist erreicht, daß bei Staubpartikeln zwischen 0,3 und 20µm langfristig die Feinstaubausfilterung, liegend etwa bei 95%, durch Zusatz des Riechstoffes keine wesentliche Minderung erfährt; beiliegendes Diagramm zeigt, daß eine Minderung im Bereich von 0,1 % ermittelt wurde, eingeschlossen den Meßfehlerbereich, der aber bei ± 1,5 % liegt. Entsprechendes ist bei aus Polycarbonat bestehenden elektrostatischen Filtern erreicht unter Berücksichtigung der diesbezüglichen Ausgestaltung der erfindungsgemäßen Lehre (s.o.).

Anlage: (I) Diagramm Vergleich des Abscheidegrades eines Electretes mit und ohne Beaufschlagung mit einem Riechstoffgemisch gemäß der Erfindung.
(II) Tabelle Geräte und Hilfsmittel zur gaschromatographischen Untersuchung.
(III) Kurve Gaschromatograph Nr. 852 I

Anlage II

## Geräte und Hilfsmittel zur gaschromatographischen Untersuchung

Gaschromatograph:    Dani 3800 ausgerüstet mit FID 681,
Temperatur Programmer 381-1,
Kontrolleinheit 380-1
Gerstel Injektor GC 11060800
Splitter

polare Trennsäule:    30 m FS-Kapillare DB-Wax (Carbowax 20 M,
chemisch gebunden)
ID 0,25 mm,
0,25 $\mu$m Polyethylenglykol-Film
Fa. ict, Best.-Nr. 122-7032

unpolare
Trennsäule:    30 m, FS-Kapillare DB - 1 (SE 30)
ID 0,25 mm
0,25$\mu$m Dimethylpolysiloxane-Film
Fa. ict, Best.-Nr. 122-1032

Injektionsspritze
Flüssigproben:    SGE 701; 10$\mu$l

Injektionsspritze
Dampfproben:    SGE 10 MA-HSV; 10ml

Brenngase:    Preßluft aus Vorwerk Netz, feintriert
Wasserstoff 3,0 (Reinheit 99,9%)
Messer Griesheim Stahlflasche

Trägergas:    Helium 4,6 (Reinheit 99,996%)
Messer Griesheim Stahlflasche

Trägergas

Reinigung:                          Oxisorb Patrone

                                    Fa. WGA, Best.-Nr. 2065


Glasflasche:                        Enghalsverpackungsglasflasche, klar

                                    500 ml mit Schraubverschluß,

                                    Fa. Behr,

                                    Best.-Nr. 120303044, Verschraubung

                                    präpariert:

                                    Schraubkappe mittig durchbohrt

                                    Ø 2 mm

                                    Dichtungsscheibe aus Gummi ausge

                                    stanzt

                                    und mit selbstklebender Alu-Folie

                                    kaschiert.


Registriergerät:                    Shimadzu Integrator C-R 1B


Magnetrührer:                       Typ RCH, Fa. Jahnke + Kunkel KG


Thermostat:                         Haake Umwälzthermostat Typ F 30


Septum für

Injektor:                           SGE Septa Typ 9 CS, Fa. WGA,

                                    Best.-Nr. 041827


**Patentansprüche**

**1.** Riechstoffgemisch für durch elektrostatisch aufgeladene Filter strömende Luft, bestehend aus einer Vielzahl von einzelnen Riechstoffen wie Methylnaphthylketon, Ambroxane, Calone, Isobutylchinaline, Dihydrojasmin oder dgl., dadurch gekennzeichnet, daß die Riechstoffe des Riechstoffgemisches jeweils ein Molekulargewicht zwischen 100 und 300 aufweisen und entweder vorwiegend polar und aprotisch oder vorwiegend apolar und aprotisch sind.

**2.** Verwendung polarer, aprotischer Riechstoffe mit einem Molekulargewicht zwischen 100 und 300, zur Anreicherung von durch elektrostatisch aufgeladene Filter auf Basis von Polypropylen strömende Luft.

**3.** Verwendung apolarer, aprotischer Riechstoffe mit einem Molekulargewicht zwischen 100 und 300, zur Anreicherung von durch elektrostatisch aufgeladene Filter auf der Basis von Polycarbonat strömende Luft.

**4.** Reinigungsmittel für eine dem Staubsaugen vorausgehende Flächenbehandlung, enthaltend ein Riechstoffgemisch bestehend auf einer Vielzahl von einzelnen Riechstoffen wie Methylnaphthylketon, Ambroxane, Calone, Isobutylchinaline, Dihydrojasmin oder dgl., dadurch gekennzeichnet, daß die Riechstoffe des Riechstoffgemischs jeweils ein Molekulargewicht zwischen 100 und 300 aufweisen und entweder vorwiegend polar und aprotisch oder vorwiegend apolar und aprotisch sind.

**Claims**

1. Aromatic substance mixture for air flowing through electrostatically charged filters, consisting of a plurality of individual aromatic substances, such as methylnaphthylketone, ambroxane, calone, isobutylquinaline, dihydrojasmine or the like, characterised in that the aromatic substances of the aromatic substance mixture each have a molecular weight between 100 and 300 and are either predominantly polar and aprotic or predominantly apolar and aprotic.

2. Use of polar, aprotic aromatic substances having a molecular weight between 100 and 300 for enriching air flowing through electrostatically charged filters based on polypropylene.

3. Use of apolar, aprotic aromatic substances having a molecular weight between 100 and 300 for enriching air flowing through electrostatically charged filters based on polycarbonate.

4. Cleaning agent for surface treatment preceding vacuuming, containing an aromatic substance mixture consisting of a plurality of individual aromatic substances, such as methylnaphthylketone, ambroxane, calone, isobutylquinaline, dihydrojasmine or the like, characterised in that the aromatic substances of the aromatic substance mixture each have a molecular weight between 100 and 300 and are either predominantly polar and aprotic or predominantly apolar and aprotic.

**Revendications**

1. Mélange de matières odorantes pour un courant d'air traversant un filtre chargé électrostatiquement, consistant en une pluralité de matières odorantes isolées, comme méthylnaphtylcétone, ambroxane, calone, isobutylquinaline, dihydrojasmin ou analogues, caractérisé en ce que les matières odorantes de ce mélange présentent chacune un poids moléculaire de 100 à 300 et sont, soit principalement polaires et aprotiques, soit principalement apolaires et aprotiques.

2. Application de matières odorantes polaires, aprotiques ayant un poids moléculaire entre 100 et 300 à l'enrichissement d'un courant d'air traversant un filtre chargé électrostatiquement à base de polypropylène.

3. Application de matières odorantes polaires, aprotiques ayant un poids moléculaire entre 100 et 300 à l'enrichissement d'un courant d'air traversant un filtre chargé électrostatiquement à base de polycarbonate.

4. Produit de purification pour un traitement de surfaces précédant l'aspiration de poussières, contenant un mélange de matières odorantes isolées, comme méthylnaphtylcétone, ambroxane, calone, isobutylquinaline, dihydrojasmin ou analogues, caractérisé en ce que les matières odorantes de ce mélange présentent chacune un poids moléculaire de 100 à 300 et sont, soit principalement polaires et aprotiques, soit principalement apolaires et aprotiques.

FIG. 1

EP 0 320 625 B1

Diagramm Vergleich Abscheidegrad

Electret mit und ohne Riechstoffgemischbeaufschlagung

EP 0 320 625 B1

**y-axis:** Abscheidegrad [%] — 0, 10, 20, 30, 40, 50, 60, 70, 80, 90, 100

mit Riechstoff: 94,87

ohne Riechstoff: 94,78

F-45- Dampf
START 04.10.14.10.

GC 852I

isotherm 120 ℃

0.5

1.73
2.14

Anlage Ⅲ

19 787 Pat-Anm. -DE-

3.25
3.58

3.07

4.36  4.45

4.04

4.84

STOP

06 ml F45 Dampf ohne Electretfilter

C-R1B
SMPL #        00
FILE  #         4
REPT #      2121
METHOD        41

| TIME | CONC | MK | AREA |
|---|---|---|---|
| 2.14 | 9.2391 | V | 4562 |
| 3.25 | 5.7637 |  | 2845 |
| 3.58 | 7.1974 | V | 3553 |
| 4.04 | 70.2384 | V V | 34681 |
| 4.36 | 2.1815 | V V | 1077 |
| 4.45 | 1.6201 | V | 799 |
| 4.84 | 3.7595 |  | 1856 |
| TOTAL | 99.9999 |  | 49377 |

F-45-Dampf
UND-ELE-Filter
START 04.10.14.18.

isotherm 120 ℃

1.26

2.13

3.31
3.61

4.06

4.44

4.87

STOP

0'6 ml F45 Dampf durch Electretfilter

C-R1B
SMPL #        00
FILE  #         4
REPT #      2122
METHOD        41

| TIME | CONC | MK | AREA |
|---|---|---|---|
| 2.13 | 27.1234 |  | 5392 |
| 3.31 | 6.0262 |  | 1198 |
| 3.61 | 5.148 | V | 1023 |
| 4.06 | 52.4588 |  | 10429 |
| 4.44 | 3.1335 |  | 623 |
| 4.87 | 6.1097 |  | 1214 |
| TOTAL | 99.9999 |  | 19881 |